# EUROPEAN PATENT SPECIFICATION

(11) **EP 0 485 232 B1**
(45) Date of publication and mention of the grant of the patent: **05.06.1996**
(21) Application number: 91310359.4
(22) Date of filing: 08.11.1991
(51) Int. Cl.: C07H 19/20, A61K 31/70

(54) **Neovascularisation inhibitors**
Neovascularisationsinhibitoren
Inhibiteurs de néovascularisation

(30) Priority: 08.11.1990 JP 305688/90
(43) Date of publication of application: 13.05.1992
(73) Proprietor: UNITIKA LTD., Amagasaki-shi Hyogo (JP)
(72) Inventor: Kawanaka, Satoshi, c/o Unitika Ltd., Uji-shi, Kyoto (JP); Dombou, Munehiki, c/o Unitika Ltd., Uji-shi, Kyoto (JP); Nakajima, Hiroshi, c/o Unitika Ltd., Uji-shi, Kyoto (JP)
(74) Representative: Cockbain, Julian, Dr.

(56) References cited:
- EP-A- 0 230 023
- EP-A- 0 246 654
- EP-A- 0 275 204
- EP-A- 0 295 037
- US-A- 4 169 011
- CHEMICAL ABSTRACTS, vol. 86, 1977, Columbus, Ohio, US; abstract no. 116656Y, J.P.HORWITZ: 'Studies on Bovine Adrenal Estrogen Sulfotransferase III. Facile Synthesis of 3'-phospho- and 2'-phosphoadenosine 5'-phosphosulfate' page 173 ;column 1 ;

## Description

The present invention relates to neovascularisation inhibiting phosphosulfate adenosines, especially adenosine 5'-phosphosulfates and salts and esters thereof. The phosphosulfate adenosines inhibit neovascularisation, and so could be used to combat malignant tumors and diseases and conditions caused by vascular hyperplasia.

Progress or metastasis of malignant tumors, and other diseases such as, for example, rheumatoid arthritis, retinopathy caused by diabetes or premature birth, senile macular or fovea degeneration, neovascular glaucoma and hypergeneration of scar tissue (formed after wound healing) are known to be caused by vascular hyperplasia (particularly hyperplasia of peripheral capillary blood vessels).

Preventive or therapeutic medicines have been developed to combat these diseases and comprise, as effective or active ingredients, substances able to inhibit neovascularisation.

The neovascularisation inhibitory substances reported to date include, for example, medroxyprogresterone (Ashiya, et al.: Int. J. Cancer, 1989, 44, 895), sulfated protamine (Ogawa, et al: Exp. Pathol., 1986, 30, 143), a combination of heparin and cortisone (J. Folkman, et al.: Science, 1983, 221, 719), prednisolone acetate (J.B. Robin: Arch. Opthalmol., 1985, 103, 284), herbimycin A (Japanese Patent Provisional Publication No. 295,509/88), a peptide from retinal pigment epithelial cells (EP-A-213,180), sulfated polysaccharides (EP-A-246,654), and phenol derivatives (EP-A-295,037).

These neovascularisation inhibiting substances are not however totally satisfactory against the above-mentioned diseases either because the inhibitory effect on neovascularisation is insufficient or because of toxic side effects.

The object of the present invention is to provide a novel neovascularisation inhibitor achieving a safe and effective result.

Thus, viewed from on aspect, the present invention provides a neovascularisation inhibiting pharmaceutical composition comprising as an active ingredient an adenosine 5'-phosphosulphate compound of formula I 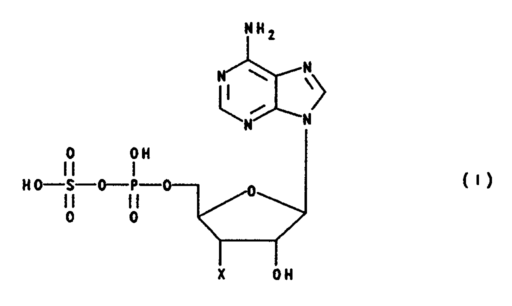
(wherein X represents a phosphate or hydroxyl group) or a physiologically tolerable ester or salt thereof together with at least one pharmaceutical carrier or excipient.

Compounds of formula I are known from the literature eg. US-A-4169011 (Horwitz). However their use as therapeutic agents is novel.

For the compositions of the invention, ester and salt forms conventional in the pharmaceutical and veterinary arts, e.g. for providing pro-drugs or enhanced solubility forms of therapeutically or prophylactically active agents, may be used. Similarly carriers and excipients conventional in these arts may be used as the pharmaceutical carriers and excipients in the compositions of the invention, compositions which may optionally contain one or more other active agents.

For use according to the invention the preferred neovascularisation inhibitors are 3-phosphoadenosine 5'-phosphosulfate (hereinafter simply referred to as "PAPS") and adenosine 5'-phosphosulfate (hereinafter simply referred to as "APS"). PAPS is a sulfate radical donor widely existing in nature, and APS is an intermediate in PAPS biosynthesis. These sulfated adenosine nucleosides are present in organisms in trace amounts. It is known that PAPS participates in, for example, the biosynthesis of chondroitin sulfate in cartilage, kermatan sulfate in skin, karatan sulfate in the cornea, heparin in mast cells, sulphatide in the brain, and phenyl sulfate and steroid sulfate in the liver and intestine.

It is thus well known that PAPS is involved in sulfation of sugar chains or proteins, however there is no record of any neovascularisation inhibitory effects of PAPS or APS, a quite novel function recognised for the first time by the present inventors. More particularly, PAPS and APS are excellent at inhibiting neovascularisation when compared to conventional neovascularisation inhibitory substances. Furthermore, the compounds used according to the invention have a very low toxicity, being natural products.

Viewed from another aspect, the present invention also provides an adenosine 5'-phosphosulphate compound of formula I or a physiologically tolerable ester or salt thereof, for use in therapy or diagnosis, particularly to treat tumour metastasis, rheumatoid arthritis, diabetic retinopathy, fovea or macular degeneration, neovascular glaucoma or vascular hyperplasia.

Viewed from a yet further aspect, the present invention also provides the use of an adenosine 5'-phosphosulphate compound of formula I or a physiologically tolerable ester or salt thereof for the manufacture of an agent in inhibiting neovascularisation, in particular to treat tumour metastasis, rheumatoid arthritis, diabetic retinopathy, fovea or macular degeneration, neovascular glaucoma or vascular hyperplasia.

Commercially available or enzyme-synthesized PAPS or APS may be employed in the preparation of the neovascularisation inhibiting compositions of the invention. Pharmaceutically acceptable salts for example with the sulfo group in salt form with alkali metal or organic counterions, e.g. potassium, sodium and ammonium salts may also be used. These salts may easily be manufactured by conventional processes.

The neovascularisation inhibitor of the present invention may be administered enterally or parenterally e.g. by injection, orally, or by a suppository, and the active agent may be administered alone or with an excipient or a carrier. The preparation of a pharmaceutical administration form may be accomplished by conventional methods: when preparing a solid oral medicine, for example, a binder, disintegrator, smoothening brightener, coloring agent, taste corrigent, and/or odor corrigent may be added as required to the active ingredient optionally together with a pharmaceutically acceptable excipient, and the resultant mixture may be processed into tablets, pills, capsules or granules by conventional pharmaceutical processes. When preparing a liquid composition a taste corrigent or a stabiliser may be added to the active ingredient to form a liquid agent which may be administered orally or by injection.

The amount of active ingredient present in the pharmaceutical composition would, of course, vary with the nature and severity of the condition to be treated, the symptoms and the actual form of medicament used, but will generally be within a range of from 0.01 to 100 w/w%, or more preferably, of from 0.05 to 80 w/w%.

The daily dosage would also depend upon the disease or disorder to be treated, frequency of administration, form of medicament, symptoms, age and body weight of the patient, but will generally be within a range of from 0.1 to 1,500 mg of active ingredient per kg of body weight, more preferably, 1 to 500 mg/kg, conveniently divided so as to permit administration two to four times during the day.

The present invention is described further below in detail by means of the following non-limiting examples.

### EXAMPLE 1

In accordance with the method of D.H. Ausprunk, et al. (Am. J. Pathol., (1975), 97, 597), the neovascularisation inhibitory effect of commercially available PAPS and APS (Sigma Corporation) was examined.

Drops containing 1, 10, 100 and 1,000 µg of PAPS or APS were added to the chorio-allantoic membranes of three-day-old fertilised chicken eggs, and the appearance of neovascularisation after two days was observed.

The results are as shown in Table 1, in which the symbols (+), (++) and (+++) show the neovascularisation inhibitory rates as compared with a control group not treated with PAPS or APS, and represent respectively 10 to 30% (+), 30 to 70% (++) and 70 to 100% (+++) (the same meanings also apply to Table 2 hereafter).

**Table 1**

| Neovascularisation inhibitory substance | Dose (µg) | | | |
|---|---|---|---|---|
| | 1 | 10 | 100 | 1,000 |
| PAPS | + | ++ | ++ | +++ |
| APS | + | + | ++ | +++ |

As is clear from Table 1, both the commercially available PAPS and APS demonstrated a slight neovascular inhibitory effect at a dose of 10 µg, and a complete neovascularisation inhibitory effect at a dose of at least 100 µg.

### EXAMPLE 2

PAPS and APS were enzyme-synthesized by using ATP sulfurylase and APS kinase derived from Penicillium chlysogenum crude enzyme solution.

More specifically, 100 g of Penicillium chlysogenum crude enzyme solution was treated with 30 to 55%-saturated ammonium sulfate fractionation, and the resultant precipitate was dialysed using 50 mM tris-hydrochloric acid buffer solution (pH8.5). Then, 10 mM magnesium sulfate, 5 mM ATP magnesium and 20 units of inorganic pyrophosphatase were mixed with this buffer solution, and the mixture was allowed to react for two hours at 30°C and then boiled. Subsequently, the supernatant thereof was passed through a Dowex 1 Column and a Sephadex G-10 Golumn, and was subjected to paper electrophoresis with Whatman 3 MM paper to obtain PAPS. APS was obtained by acid-decomposing the PAPS. (Dowex and Sephadex are registered Trade Marks.)

Then, the thus enzyme-synthesized PAPS and APS were tested for neovascularisation inhibition in accordance with the same procedure as in Example 1.

The results are as shown in Table 2: the PAPS and APS obtained by enzyme-synthesis inhibited neovascularisation in a dose-dependent manner within a dose range of from 1 to 1,000 µg, similarly to those commercially available in Example 1.

**Table 2**

| Neovascularisation inhibitory substance | Dose (µg) | | | |
|---|---|---|---|---|
| | 1 | 10 | 100 | 1,000 |
| PAPS | + | ++ | ++ | +++ |
| APS | + | + | ++ | +++ |

### EXAMPLE 3

In accordance with the method proposed by M.A. Gimbrone, et al. (J. Nat. Can. Inst., 1974, 52, 413), the inhibitory effect of commercially available PAPS and APS on neovascularisation in rabbit corneas was examined.

First, the center portion of a rabbit cornea was incised by about 2mm using a scalpel to form a spot along the corneal wall and then a previously prepared slow-releasing pellet containing 10 µg prostaglandine E₁ or 200 µg cuprous chloride and 100 µg or 1.0 mg PAPS or APS (Sigma Corporation) in compliance with the method proposed by R. Langer, et al. (Nature, 1979, 263, 797) was inserted and fixed onto this spot.

PAPS and APS were observed to inhibit completely the neovascularisation caused by prostaglandine E₁ or cuprous chloride at all doses of 100 µg and 1.0mg. The inhibition continued for the following seven days.

### EXAMPLE 4

The therapeutic effect of experimental diabetic retinopathy was investigated for a neovascularisation inhibitor prepared by dissolving PAPS or APS into 50% dimethylsulfoxide.

First, 65 mg/kg streptozocin was administered to six-week aged male Wister rats via the vena oaudalis, and approximately three months later, 3, 3′-iminodipropionitrile was administered. Rats in which abnormal growth of capillary vessel (hyperplasia retinitis) was observed were screened out and were subjected to a medicament administration test with ten rats per group.

The neovascularisation inhibitor was administered to the rats in amounts of 1.5, 3.0, 4.5 or 6.0 mg/kg for ten consecutive days by I.P. injections, and the extent of capillary vessel hyperplasia in vitro was observed. For comparison purposes, the effect of the medroxyprogesterone acetate (MPA), (a conventional substance for inhibiting the neovascularisation) was also tested by the same procedure as described above.

The results are as shown in Table 3, in which the symbol (-) indicates that no improvement in the pathological state was observed following administration of the medicament compared with rats in the control group which received 0.1 ml/10g of 50% dimethylsulfoxide for ten days. The symbols (+), (++) and (+++) indicate, respectively, an improvement in the pathological state of 10 to 30%, 30 to 70% and 70 to 100%.

As is clear from Table 3, the neovascularisation inhibitor of the present invention containing PAPS or APS as active ingredient has an improving effect on the pathological state of 10% for the 1.5 mg/kg administration group, 70% for 3.0 mg/kg group, and at least 80% for the 6.0 mg/kg group. Within any dose, an improved therapeutic effect was shown compared to conventional neovascularisation inhibitors containing MPA as an active ingredient.

**Table 3**

| Effective ingredient of neovascularisation inhibitor substance | Dose (mg/kg) | | | |
|---|---|---|---|---|
| | 1.5 | 3.0 | 4.5 | 6.0 |
| PAPS | + | ++ | +++ | +++ |
| APS | + | + | +++ | +++ |
| MPA | - | + | + | ++ |

Next, an example of an experiment regarding the toxicity of the neovascularisation inhibitors of the present invention is described.

### EXAMPLE

### Experiment to investigate the toxicity of PAPS and APS

Injections were prepared by dissolving PAPS or APS in 50% dimethylsulfoxide, and these were parenterally administered within a range of from 500 to 3,500 mg/kg (I.V. or I.P. injection) to rats.

No deaths or toxic symptoms were observed.

## Claims

1. A neovascularisation inhibiting pharmaceutical composition comprising as an active ingredient an adenosine 5'-phosphosulphate compound of formula I 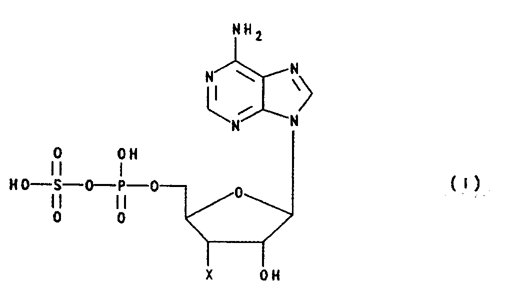 (wherein X represents a phosphate or hydroxyl group) or a physiologically tolerable ester or salt thereof together with at least one pharmaceutical carrier or excipient.

2. A composition as claimed in claim 1 containing said compound at a concentration by weight of 0.05 to 80%.

3. An adenosine 5'-phosphosulphate compound of formula I as defined in claim 1 or a physiologically tolerable ester or salt thereof for use in therapy or prophylaxis.

4. The use of an adenosine 5'-phosphosulphate compound of formula I as defined in claim 1 or a physiologically tolerable ester or salt thereof for the manufacture of an agent for use in inhibiting neovascularisation.

## Patentansprüche

1. Pharmazeutische Zusammensetzung zur Hemmung der Neovaskularisation, enthaltend als Wirkstoff eine Adenosin-5'-phosphosulfat-Verbindung der Formel I 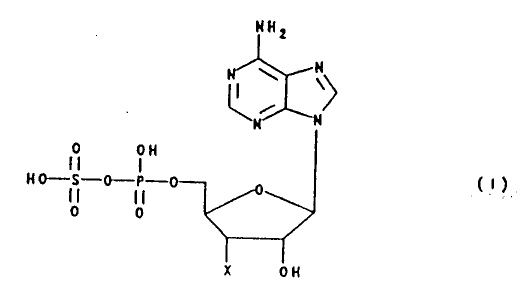 (worin X eine Phosphat- oder Hydroxylgruppe bedeutet) oder einen physiologisch verträglichen Ester oder Salz davon zusammen mit mindestens einem pharmazeutischen Trägerstoff oder Verdünnungsmittel.

2. Zusammensetzung nach Anspruch 1, enthaltend die Verbindung in einer Konzentration von 0,05 bis 80 Gew.-%.

3. Adenosin-5'-phosphosulfat-Verbindung der Formel I nach Anspruch 1 oder ein physiologisch verträglicher Ester oder Salz davon zur Verwendung bei der Therapie oder Prophylaxe.

4. Verwendung einer Adenosin-5'-phosphosulfat-Verbindung der Formel I nach Anspruch 1 oder eines physiologisch verträglichen Esters oder Salzes davon bei der Herstellung eines Mittels zur Verwendung bei der Hemmung der Neovaskularisation.

## Revendications

1. Composition pharmaceutique inhibant la néovascularisation, comprenant comme principe actif un composé adénosine 5'-phosphosulfate de formule I: 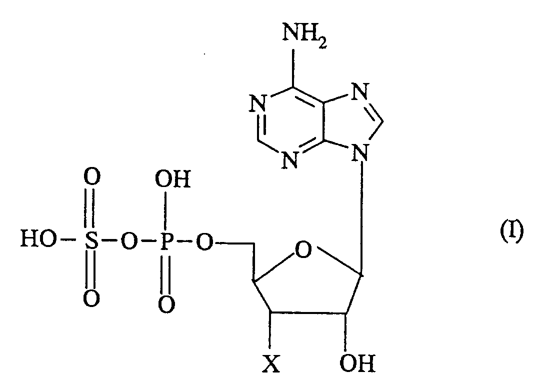 (où X représente un groupement phosphate ou hydroxyle) ou un ester ou un sel physiologiquement toléré de ce dernier, avec au moins un véhicule ou excipient pharmaceutique.

2. Composition selon la revendication 1, contenant ledit composé à une concentration en poids de 0,05 à 80 %.

3. Composé adénosine 5'-phosphosulfate selon la revendication 1 ou ester ou sel physiologiquement toléré de ce dernier pour usage thérapeutique ou prophylactique.

4. Utilisation d'un composé adénosine 5'-phosphosulfate de formule I selon la revendication 1 ou d'un ester ou d'un sel physiologiquement toléré de ce dernier pour la préparation d'un agent à utiliser pour inhiber la néovascularisation.
